# EUROPÄISCHE PATENTSCHRIFT

(11) **EP 0 641 772 B1**
(45) Veröffentlichungstag und Bekanntmachung des Hinweises auf die Patenterteilung: **05.11.1997**
(21) Anmeldenummer: 94113366.2
(22) Anmeldetag: 26.08.1994
(51) Int. Cl.: C07C 261/04, C14C 3/18

(54) **Cyanamidgruppen enthaltende Polyisocyanat-Additionsverbindungen und ihre Verwendung**
Polyisocyanato addition compounds containing cyanamide groups and their use
Composés d'addition de polyisocyanates contenant groupes de cyanamide et leur utilisation

(30) Priorität: 08.09.1993 DE 4330378
(43) Veröffentlichungstag der Anmeldung: 08.03.1995
(73) Patentinhaber: BAYER AG, 51368 Leverkusen (DE)
(72) Erfinder: Träubel, Dr. Harro, D-51373 Leverkusen (DE); Münzmay, Dr. Thomas, D-41539 Dormagen (DE); Hassel, Dr. Tillmann, D-51061 Köln (DE); Krümmel, Wilhelm, D-51375 Leverkusen (DE); Ehlert, Hans-Albert, D-51373 Leverkusen (DE); Kochta, Dr. Joachim, D-53119 Bonn (DE)

(56) Entgegenhaltungen:
- EP-A- 0 185 184
- EP-A- 0 312 836
- EP-A- 0 538 649

## Beschreibung

Die Erfindung betrifft Umsetzungsprodukte aus
a) organischem Polyisocyanat,
b) pro Äquivalent in a) enthaltenem NCO oder latentem NCO 0,005 bis 0,9 Äquivalente C₆-C₂₄-, vorzugsweise C₁₂-C₁₈-Alkohol,
c) pro Äquivalent in a) enthaltenem NCO oder latentem NCO 0,1 bis 0,995 Mol Cyanamid und
d) Ammoniak oder flüchtigem Amin als Neutralisationsmittel für die Cyanamidgruppen,
wobei diese Umsetzungsprodukte frei von Polyester- und Polyethergruppen und frei von Halogenatomen sind.

Der Begriif "Cyanamidgruppen" im Sinne der Erfindung umfaßt funktionelle Gruppen jeglicher Art, sofern sie Gruppierungen der Formel 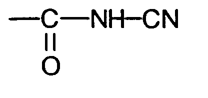 (also z.B. Cyanharnstoffgruppen 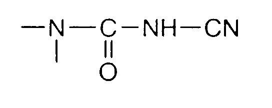 oder Cyanaminocarbonylharnstoffgruppen 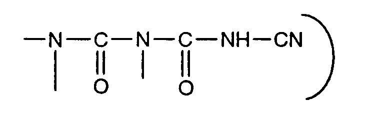 enthalten, und die Salze dieser Verbindungen enthaltend Gruppierungen der Formel

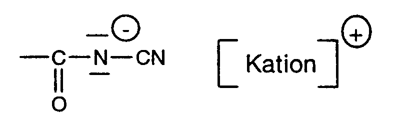

Die Verwendbarkeit von anionisch modifizierten Polyisocyanat-Additionsprodukten zur Herstellung wäßriger Dispersionen beruht auf der Anwesenheit eingebauter ionischer Zentren, insbesondere eingebauter Sulfonat- oder Carboxylatgruppen. Bei der Herstellung von Beschichtungen aus diesen Dispersionen verbleiben im allgemeinen die ionischen Zentren in den resultierenden Beschichtungen, was eine Verminderung der Wasserresistenz der resultierenden Beschichtung zur Folge hat.

Weiterhin sind in Wasser dispergierbare Polyisocyanat-Additionsprodukte bekannt, die durch anionische Cyanharnstoffgruppen modifiziert sind (vergl. DE-OS 3 441 934, 3 600 595, 3 735 198, 3 813 840 und 4 133 572). Aus derartigen Dispersionen erhaltene Beschichtungen sind wesentlich wasserresistenter als aus üblichen PUR-Dispersionen erhaltene Beschichtungen, weil die hydrophilierenden Cyanharnstoffanionen nach Verlust des Gegenions selbstvernetzenden Charakter erhalten und dadurch nach erfolgter Vernetzung ihre Hydrophilie verlieren.

Die Resistenz gegen Wasser bzw. die Reduzierung einer den Produkten inhärent zu eigenen Hydrophilie ist auch bei Hilfsmitteln zum Dispergieren (z.B. von Paraffinen, Pigmenten, Polymeren) wichtig.

Während Gerbstoffe chemische Bindungen zum Kollagen des Leders ausbilden und durch diese Vernetzung die Schrumpfungstemperatur des Leders anheben sollen, handelt es sich bei den sogenannten Nachgerbstoffen um Hilfsmittel, die selbst keine oder nur geringe gerbende Wirkung besitzen, aber zu besserer Färbbarkeit und höherer Weichheit und Fülle führen. Sie müssen in der Lage sein, in das Leder einzudringen und dort u.a. Fettungsmittel oder vegetabile Gerbstoffe zu dispergieren und zu fixieren. Beispiele solcher Nachgerbstoffe umfassen Formaldehyd/Naphthalinsulfonsäure-Kondensationsprodukte, (Meth-)Acrylsäure-Polymerisate, (Meth-)Acrylsäure/Acrylnitril-Copolymerisate, Oligourethane, enthaltend ionische Gruppen (s. z.B. US 4 106 897) und Dicyandiamid-Harze (JALCA 83 (1988), 196).

Überraschenderweise wurde nun gefunden, daß bei der Verwendung der eingangs definierten Umsetzungsprodukte als Nachgerbstoffe sehr weiche Leder mit hervorragendem Griff entstehen und die Umsetzungsprodukte bis zu einer praktisch quantitativen Baderschöpfung in das Leder einziehen. Die gewünschten Echtheiten werden überraschend schnell erzielt, so daß die Leder ohne unerwünschte Verzögerung der weiteren Verarbeitung zugeführt werden können. Ein wesentlicher Vorteil der Produkte besteht darin, daß sie im Leder zu einem wasserunlöslichen (nicht hydrophilen) Dimerisat oder Oligomerisat reagieren und folglich weder ausgewaschen werden noch nenneuswert migrieren, sobald diese Reaktion abgeschlossen ist.

Gegenstand der Erfindung sind also die eingangs definierten Umsetzungsprodukte. Sie besitzen vorzugsweise mittlere Molekulargewichte (Zahlenmittel) von unter 5 000, vorzugsweise von 800 bis 3 000.

Weiterer Gegenstand der Erfindung ist ein Verfahren zur Herstellung dieser Produkte durch Umsetzung der Komponenten a) bis d).

Für die Herstellung der erfindungsgemäßen Umsetzungsprodukte sind als organische Polyisocyanate a) beliebige organische Verbindungen geeignet, die mindestens zwei freie Isocyanatgruppen pro Molekül aufweisen. Vorzugsweise werden Diisocyanate X(NCO)₂ eingesetzt, wobei
- X: für einen zweiwertigen aliphatischen Kohlenwasserstoffrest mit 4 bis 12 Kohlenstoffatomen, einen zweiwertigen cycloaliphatischen Kohlenwasserstoffrest mit 6 bis 15 Kohlenstoffatomen, einen zweiwertigen aromatischen Kohlenwasserstoffrest mit 6 bis 15 Kohlenstoffatomen oder einen zweiwertigen araliphatischen Kohlenwasserstoffrest mit 7 bis 15 Kohlenstoffatomen steht.

Beispiele derartiger bevorzugt einzusetzender Diisocyanate sind Tetramethylendiisocyanat, Methylpentamethylendiisocyanat, Hexamethylendiisocyanat, Dodecamethylendiisocyanat, 1,4-Diisocyanato-cyclohexan, 1-Isocyanato-3,3,5-trimethyl-5-isocyanato-methyl-cyclohexan, 4,4'-Diisocyanato-dicyclohexyl-methan, 4,4'-Diisocyanato-dicyclohexylpropan-(2,2), 1,4-Diisocyanatobenzol, 2,4-Diisocyanatotoluol, 2,6-Diisocyanatotoluol, 4,4'-Diisocyanato-diphenylmethan, 2,2'- und 2,4'-Diisocyanato-diphenylmethan, p-Xylylendiisocyanat, p-Isopropyliden-diisocyanat, sowie aus diesen Verbindungen bestehende Gemische.

Es ist selbstverständlich auch möglich, die in der Polyurethanchemie an sich bekannten höherfunktionellen Polyisocyanate oder auch an sich bekannte modifizierte, beispielsweise Carbodiimidgruppen, Allophanatgruppen, Isocyanuratgruppen, Urethangruppen und/oder Biuretgruppen aufweisende Polyisocyanate alleine oder anteilig (mit)zuverwenden.

Weiterhin geeignete Polyisocyanate a) sind solche Verbindungen, die Oxadiazintrion-Struktureinheiten (I) oder Uretdion-Struktureinheiten (II) 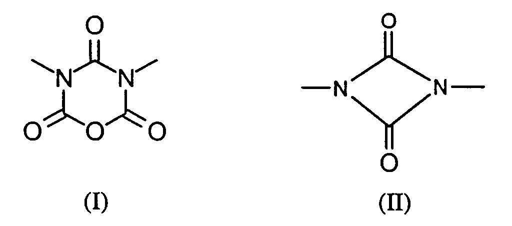 enthalten.

Als Aufbaukomponenten a) zur Einführung der Oxadiazinthon- und/oder Uretdion-Struktureinheiten der Formeln (I) bzw. (II) eignen sich Diisocyanate der Formeln (III) und/oder (IV) 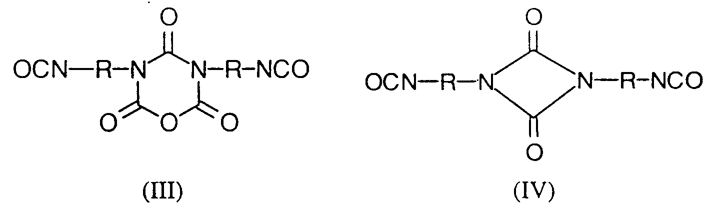 worin
- R: unabhängig voneinander für den zweiwertigen Rest eines aliphatischen Kohlenwasserstoffs mit 1 bis 15 C-Atomen, eines cycloaliphatischen Kohlenwasserstoffs mit 3 bis 15 C-Atomen, eines araliphatischen Kohlenwasserstoffs mit 7 bis 15 C-Atomen oder eines aromatischen Kohlenwasserstoffs mit 6 bis 12 C-Atomen steht.

Beispiele für derartige Polyisocyanate a) sind 1,3-Bis-(5-isocyanato-1,3,3-trimethyl-cyclohexyl-methylen)-2,4-dioxo-1,3-diazetidin; 1,3-Bis-(3-isocyanato-4-methylphenyl)-2,4-dioxo-1,3-diazetidin; 1,3-Bis-(6-isocyanato-hexyl)-2,4-dioxo-1,3-diazetidin; 3,5-Bis-(5-isocyanato-1,3,3-trimethyl-cyclohexyl-methylen)-2,4,6-trioxo-tetrahydro-1,3,5-oxadiazin; 3,5-Bis-(4-isocyanato-cyclohexyl)-2,4,6-trioxo-tetrahydro-1,3,5-oxadiazin und 3,5-Bis-(6-isocyanato-hexyl)-2,4,6-trioxo-tetrahydro-1,3,5-oxadiazin (Desmodur® LB 202, Bayer AG).

Von den Isocyanaten der Formeln (III) und (IV) werden diejenigen der Oxadiazintrion-Reihe (III) bevorzugt eingesetzt, besonders bevorzugt 3,5-Bis-(6-isocyanato-hexyl)-2,4,6-trioxo-tetrahydro-1,3,5-oxadiazin.

Da man durch Umsetzung mit dem Alkohol Isocyanatgruppen verliert, sind Isocyanate mit mehr als 2 Isocyanatgruppen ganz besonders bevorzugt. Zu diesen Isocyanaten zählen die Isocyanurate, Biurete, Trisurethane (und deren höherfunktionelle Analoga) des TDI, IPDI, MDI, HDI, H12-MDI, 1,4-Diisocyanato-cyclohexans etc. Wegen der besseren Lichtechtheiten sind auch hier die aliphatischen Isocyanate ganz besonders bevorzugt.

Für die Herstellung der erfindungsgemäßen Umsetzungsprodukte geeignete Alkohole b) umfassen beispielsweise Alkanole wie n-Hexanol, n-Octanol, 2-Ethylhexanol, Laurylalkohol, Cetylalkohol, Stearylalkohol, Alkenole wie Oleylalkohol, cycloaliphatische Alkohole wie Cyclohexanol, Hydroxymethylcyclohexan, araliphatische Alkohole wie Benzylalkohol.

Das für die Herstellung der erfindungsgemäßen Umsetzungsprodukte zu verwendende Cyanamid c) wird vorzugsweise in Salzform, die durch Neutralisation mit der Komponente d) entsteht, eingesetzt. Als Kation werden bevorzugt die Kationen flüchtiger Amine eingesetzt. Die flüchtigen Amine d) umfassen tertiäre Amine mit bis zu 12 C-Atomen pro Molekül, wie z.B. Triethylamin, Trimethylamin, Triisopropylamin, Tri-n-butylamin, N,N-Dimethylaminoethanol, Trisisopropanolamin, Pyridin oder N-Methylmorpholin. Ammoniak und Triethylamin sind ganz besonders bevorzugte Komponenten d).

Im allgemeinen stellt man durch Umsetzung der Komponenten a) und b) zunächst ein Vorprodukt her, das dann im Wasser in Gegenwart von Cyanamid c) und Neutralisationsmittel d) oder in Gegenwart eines mit d) neutralisierten Cyanamids zum gewünschten Endprodukt umgesetzt wird. Die Reaktionstemperatur für diese Reaktionen wird im allgemeinen zwischen 20 und 80°C, vorzugsweise zwischen 30 und 60°C gehalten.

Die Reaktionszeit beträgt im allgemeinen zwischen wenigen Minuten und einigen Stunden. Im Falle der bevorzugten Oxadiazintrionstrukturen ist der Reaktionsverlauf leicht zu verfolgen. Die Reaktion ist abgeschlossen, wenn die Kohlendioxidentwicklung beendet ist.

Die Festkörpergehalte der entstehenden wäßrigen Dispersionen können in weiten Grenzen schwanken, liegen aber in der Regel bei 5 bis 70, vorzugsweise 20 bis 60 Gew.-%.

Nach einer bevorzugten Ausführungsform werden bis zu 0,9 Äquivalente, insbesondere bis zu 0,7 Äquivalente der freien Isocyanatgruppen der Polyisocyanate a) mit Alkohol b) umgesetzt; die restlichen Isocyanatgruppen werden dann mit den Komponenten c) und d) weiter umgesetzt. Falls gewünscht, kann man die Reaktion auch so führen, daß die Komponenten c) und d) im Unterschuß eingesetzt werden - die dadurch übrigbleibenden NCO-Gruppen reagieren dann ab.

Nach einer besonders bevorzugten Ausführungsform werden die freien Isocyanatgruppen des Diisocyanatohexyl-oxadiazintrions mit 0,05 bis 1 Äquivalenten Alkohol b) urethanisiert und anschließend der Oxadiazintrion-Ring, der eine latente NCO-Gruppe im Sinne dieser Erfindung darstellt, mit Cyanamid oder neutralisiertem Cyanamid gesprengt.

Die erfindungsgemäßen Umsetzungsprodukte besitzen folgende Eigenschaften:
- beim Auftrocknen und Abgeben ihres Gegenions dimerisieren oder oligomerisieren die Verbindungen und verlieren ihren ionischen Charakter;
- damit verlieren sie auch ihre dispergierenden Eigenschaften; damit dispergierte Polymere werden ausgefällt;
- sofern sie Reste längerkettiger Alkohole b) besitzen, können sie hydrophobe Charaktereigenschaften entwickeln;
- sofern die Produkte mit Dispersionen, wie sie z.B. in DE-OS 41 33 572 beschrieben sind und die Cyanamid als hydrophilierende Gruppen enthalten, zusammen verwandt werden, wirken sie wie Vernetzer.

Die erfindungsgemäßen Umsetzungsprodukte eignen sich zur Verwendung in folgenden Einsatzgebieten:
- weichmachende und gegebenenfalls hydrophobierende Nachgerbung von Leder;
- Imprägnierungen von Textilien zur waschbeständigen Verbesserung von Griff und Aussehen bzw. Einsatz im Textildruck;
- Verstärkung der Hydrophobierwirkung von hydrophobierenden Substanzen, z.B. von Paraffin;
- als Dispersionsstabilisator für Textil- und Lederhilfsmittel;
- als Emulgator für Celluloseester (wie Nitrocellulose, Celluloseacetobutyrat), PVC, Polyvinylacetat, Polyamid, Polyurethan, Polyethylen, Polypropylen, wie sie z.B. als Oberflächenbehandlungsmittel harter Teile (ABS, PVC) oder weicher Substrate (Leder, Textilien, Papier) verwendet werden.

Die Einsatzmengen der erfindungsgemäßen Umsetzungsprodukte liegen gewöhnlich im Bereich von 1 bis 500, vorzugsweise 20 bis 200 Gew.-%, bezogen auf Festkörper sowohl der angewendeten Umsetzungsprodukte als auch des Substrats.

Werden die erfindungsgemäßen Umsetzungsprodukte als Emulgatoren für Polymere eingesetzt, die in nicht mit Wasser mischbaren organischen Lösungsmitteln dispergiert sind, können sie in Mengen von 5 bis 100 Gew.-% (festes Umsetzungsprodukt) pro Lösung des Polymeren eingesetzt werden.

Wenn man die erfindungsgemäßen Umsetzungsprodukte nur als Emulsionsvermittler nutzen möchte, genügt im allgemeinen die Verwendung von 1 bis 20, vorzugsweise 3 bis 10 Gew.-% (festes Umsetzungsprodukt) pro zu emulgierende Dispersion.

Die mit den erfindungsgemäßen Umsetzungsprodukten versetzten Mischungen können in einfacher Weise (durch Rühren oder Eingießen in die Anwendungsflotte) in eine Dispersion überführt werden. Im Falle der Ledernachgerbung wird im allgemeinen lediglich in die Nachgerbflotte gegossen; durch die Drehbewegung des Fasses oder der Gerbmaschinen wird die Dispersion aufgenommen. Der pH-Wert der Nachgerbflotte bzw. des Leders sollte oberhalb 3, besser oberhalb von 5 liegen. Selbstverständlich kann man noch synthetische Gerbstoffe, Polymergerbstoffe oder mineralische Gerbstoffe, Fettungsmittel und Farbstoffe hinzufügen. Durch Zugabe von Säure kann man die Produkte gezielt dimerisieren bzw. oligomerisieren.

Im Falle der Oberflächenbehandlung wird die Dispersion durch Spritzen, Gießen, Drucken, Rakeln direkt auf die zu behandelnde Oberfläche gebracht oder im Umkehrverfahren erst auf einen einstweiligen Träger aufgebracht, gegebenenfalls eine oder mehrere weitere Schichten darauf aufgebracht, das zu kaschierende Substrat damit verhaftet und anschließend vom einstweiligen Träger abgezogen.

Bei Textilien bringt man die erfindungsgemäßen Umsetzungsprodukte - neben den oben geschilderten Verfahren - vor allem durch einen Foulard-Prozeß auf.

Weiterer Gegenstand der Erfindung ist also die Verwendung der erfindungsgemäßen Umsetzungsprodukte zur Nachgerbung von Leder, als Leimungsmittel für Papier, als Ausrüstmittel für Leder und Textilien und als Emulgatoren.

Die Prozentangaben der nachfolgenden Beispiele beziehen sich jeweils auf das Gewicht.

### Beispiele

### Beispiel 1

In einem 1 l-Dreihalskolben werden 186,4 g (1 mol) Laurylalkohol und 22 g (1 mol) Isophorondiisocyanat 6 h bei 60°C gerührt. Das Reaktionsgemisch hat einen Isocyanatgehalt von 10,28 %.

In das Reaktionsgemisch werden 42 g (1 mol) Cyanamid eingetragen und 30 min nachgerührt. Bei 60°C werden 101,2 g (1 mol) Triethylamin portionsweise zugegeben. Nach 1 h Nachrührzeit wird in eine flache Form gegossen. Man erhält eine bei Raumtemperatur glasartig erstarrende Masse, die gut wasserlöslich ist

### Beispiel 2

In einem 1 l-Dreihalskolben werden 373 g (2 mol) Laurylalkohol und 444 g (2 mol) Isophorondiisocyanat bei 60°C gerührt. Nach 6 h hat das Reaktionsgemisch einen Isocyanatgehalt von 10,36 %.

Das Reaktionsprodukt wird bei Raumtemperatur langsam unter Rühren in eine Losung von 82 g (2 mol) Cyanamid und 202,2 g (1,96 mol) Triethylamin in 490 g Wasser eingegossen. Man erhält eine leicht opake Lösung, die mit weiteren 400 g Wasser auf einen Feststoffgehalt von 46 % eingestellt wird.

### Beispiel 3

In einem 1 l-Dreihalskolben werden 380,6 g (2,04 mol) Laurylalkohol und 932,4g (4,20 mol) Isophorondiisocyanat 4 h bei 70°C gerührt. Das Gemisch hat einen Isocyanatgehalt von 20 %.

Das Reaktionsprodukt wird langsam in eine Lösung von 252 g (6 mol) Cyanamid und 607 g (6 mol) Triethylamin in 3 250 g Wasser eingegossen. Man erhält eine wäßrige Lösung mit einem Festkörpergehalt von 39 %.

### Beispiel 4

In einem 1 l-Dreihalskolben werden 402 g (1,5 mol) 9-Octadecen-1-ol und 315 g (0,75 mol) 3,5-Bis-(6-isocyanatohexyl)-2,4,6-trioxo-tetrahydro-1,3,5-oxadiazin (technisches Produkt, MG = 200) bei 90°C gerührt.

Nach 5 h ist die Reaktionsmischung isocyanatfrei. Es werden 63 g 50 %ige wäßrige Cyanamidlösung (0,75 mol) und 77,3 g Triethylamin bei 50°C langsam zugetropft. Nach vollständiger Zugabe wird 2 h bei 50°C nachgerührt

### Beispiel 5

Wiederholung des Beispiels 3 mit folgenden Änderungen:

1 mol Oleylalkohol, 1 mol 3,5-Bis-(6-isocyanatohexyl)-2,4,6-trioxo-tetrahydro-1,3,5-oxydiazin, 1,35 mol Triethylamin und 1,5 mol Cyanamid. Man erhält eine 30,6 %ige wäßrige Dispersion.

### Beispiel 6

In einem 1 l-Dreihalskolben werden 611,5 g eines biuretisierten Hexamethylendiisocyanates (Desmodur N 3200, Bayer AG) und 248,7 g Oleylalkohol 4 h bei 60°C gerührt. Das Reaktionsprodukt hat einen Isocyanatgehalt von 10,74 %.

790 g des Reaktionsprodukts werden langsam in eine Losung von 83,3 g Cyanamid und 200,7g Triethylamin in 1 000 g Wasser eingerührt. Man erhält eine wäßrige Lösung mit einem Festkörpergehalt von ca. 50 %.

### Beispiel 7

### analog Beispiel 1 der DE-OS 3 813 840

In einem 5 l-Planschlifftopf werden 1 000 g eines Hexandiol-Neopentylglykol-Mischadipates (OH-Z = 65) mit 2 155 g eines biuretisierten Hexamethylendiisocyanates (Desmodur N 3200, Bayer AG) zur Reaktion gebracht (NCO = 14 %).

2 450 g dieses Prepolymers werden in eine Lösung von 345 g Cyanamid und 730 g N,N-Dimethylethanolamin in 1 500 g Wasser unter Rühren zugetropft. Man erhält eine Dispersion mit einem Feststoffgehalt von 70 %.

### Beispiel 8

In einem 1,5 l-Dreihalskolben werden 571,2 g Hexamethylendiisocyanat (Desmodur H, Bayer AG), 134 g Trimethylolpropan und 348,2 g Oleylalkohol 6 h bei 80°C gerührt. Das fertige Prepolymer hat einen Isocyanatgehalt von 10 %. 800 g des Isocyanatprepolymeren werden langsam in eine Losung von 80g Cyanamid und 192,8 g Triethylamin in 2 100 g Wasser eingerührt. Man erhält eine wäßrige Losung mit einem Festkörpergehalt von ca. 34 %.

### Beispiel 9

In einem 2 l-Dreihalskolben werden 888 g Hexamethylendiisocyanat (Desmodur H, Bayer AG), 104 g Trimethylolpropan, 442,3 g eines Dimethylol-poly-dimethyl-siloxans mit einem Gehalt von ca. 6 % Hydroxylgruppen (Baysilon OF-OH, Bayer AG) und 565 g Oleylalkohol 5 h bei 90°C gerührt. Das fertige Prepolymer hat einen Isocyanatgehalt von 9,1 %. 1 000 g des Isocyanatprepolymeren werden langsam in eine Losung von 85,6 g Cyanamid und 185,6 g Triethylamin in 1 270 g Wasser eingerührt. Man erhalt eine wäßrige Lösung mit einem Festkörpergehalt von ca. 50 %.

### Anwendungsbeispiel 1

### Einsatz bei der Nachgerbung von Leder.

Ein ca. DIN A 5 großes Stück wet-blue wurde in einer Gerbflasche mit 100 % Wasser versetzt, durch Zugabe von 1 % Natriumformiat und 0,2 % Natriumbicarbonat (1 h Laufzeit) wurde der pH-Wert auf 4,9 eingestellt Sodann wurden 11 g (3 % Wirksubstanz auf wet-blue) des Produktes aus Beispiel 5 zugegeben und 5 h laufen lassen. Die Flotte zehrte völlig aus und der pH-Wert blieb konstant bei 4,8. Beim Trocknen entstand ein weiches, volles Leder.

Mit ähnlich guten Ergebnissen wurden die Produkte der Beispiele 1 bis 4 eingesetzt. Auch die Mitverwendung von klassischen Fettungs- und Nachgerbstoffen war möglich, sofern der pH-Wert der Leder bzw. der Flotte oberhalb von 4 lag.

### Anwendungsbeispiel 2

### als Hydrophobier- Und Weichmachungsmittel für Leder

150 Teile des Produktes aus Beispiel 5 wurden mit 22,5 Teilen Diisopropylnaphthalin und 22,5 Teilen eines technischen Paraffins (C₁₄ - C₁₇) sowie 50 Teilen Paraffin (C₂₀ - C₂₇) homogen emulgiert. Es entstand eine 37 %ige wäßrige Dispersion.

100 kg chromgegerbte Rindhäute der Falzstärke 1,7 mm wurden gemaß der Bayer-Anwendungsvorschrift G 903 10 Minuten mit 200 % Wasser gewaschen. Dann wurde mit 50 % Wasser (40°C) und 2 % TANIGAN PF-N (neutralisierender Nachgerbstoff der Bayer AG, Leverkusen) 40 min lang gehandelt; dabei stellte sich ein pH-Wert von 4,7 ein. Normalerweise hätte man jetzt mit 4 % Farbstoff gefärbt, was in diesem Beispiel unterblieb. Man versetzte mit 3 % BAYTIGAN AR (Nachgerbstoff aus 40 %iger wäßriger Polyacrylsäure der Bayer AG, der zuvor mit der dreifachen Menge Wasser vorverdünnt worden war) und gab nach 20 min eine Mischung aus 2 % CORIPOL BZN (Fettungsmittel der Stockhausen GmbH, Krefeld) und 98 % der oben hergestellten, erfindungsgemäßen Mischung in der 4-fachen Wassermenge verdünnt dazu und ließ weitere 20 min laufen. 2 % TANIGAN OS (synthetischer Austauschgerbstoff, Bayer AG, Leverkusen) und 3 % gesüßte Kastanie wurden zugefügt und nach 90 min die Flotte (eine pH-Wertes von 4.8) abgelassen. Danach erfolgte ein Waschvorgang mit 200 % Wasser von 50°C. Sodann wurde die Hauptmenge der oben hergestellten Mischung von 5 % in 50 % 60°C warmem Wasser zugefügt und nach 75 min die Flotte abgelassen. Man spülte nochmals mit 200 % Wasser (50°C) 10 min, entnimmt die Leder, legt sie über Nacht auf Bock, reckt aus und trocknet im Vakuum und schließlich hängend aus. Dann wurden die Leder gestollt und 30 sek auf der Vakuumpumpe abgebügelt. Die Leder waren weich und hydrophob (aufgespritztes Wasser perlte ab).

### Anwendungsbeispiel 2.1

### Anwendung dieser selbstinhibierenden Emulgatoren als Lederfettungsmittel

Das in Beispiel 6 hergestellte Produkt wurde 1:1 mit Rizinusöl unter Zuhilfenahme von 0,5 % Ammoniak emulgiert Die daraus entstehende Emulsion wurde anschließend auf Wet Blue eingesetzt. Dabei wurden 150 g chromgegerbtes Leder in 100 % Wasser von 40°C gelegt. In einer Flasche wurde mit 1 % Natriumformiat und 0,2 % Natriumbicarbonat 1 Stunde laufengelassen, bis sich der pH auf 4,6 einstellte. Man verwarf die Flotte und gab in neuer Flotte von 100 % Wasser (50°C) von der Mischung aus Emulgator und Rizinusöl 2,5 % (Trockensubstanz) zu. Man ließ 2 Stunden laufen. Der End-pH betrug 6,0. Danach wusch man kurz, trocknete das Leder und stollte es, um es beurteilen zu können. Das so behandelte Leder war weich und zeigte einen deutlichen Fettungseffekt. Analoge Versuche wurden mit Sonnenblumenöl, Rapsöl bzw. Rindertalg durchgeführt. In allen diesen Fällen erhielt man durch die Behandlung mit dem selbstinhibierenden Emulgator zusammen mit der Fettkomponente ein weiches, gefettetes Leder.

### Anwendungsbeispiel 2.2

### Vergleich zu Stand der Technik

Analog zum Anwendungsbeispiel 2 wurden 150 Teile des Produktes aus Beispiel 7 mit 22,5 Teilen Diisopropylnaphthalin und 22,5 Teilen C₁₄₋₁₇-Paraffin sowie 50 Teilen C₂₀₋₂₇-Paraffin emulgiert.

Man ließ diese Emulsion 24 Stunden bei Raumtemperatur stehen. Im Gegensatz zu der des Anwendungsbeispiels 2 separierte sie. In der Nachgerbung/Hydrophobierung zeigte sie fast keinen Effekt.

### Anwendungsbeispiel 3

### als Dispergator für Celluloseacetobutyrat als Finish für Leder

70 g Celluloseacetobutyrat werden in 340 g i-Octylacetat und 189 g Butylacetat unter Rühren aufgelöst. Man fügt 35 Teile Weichmacher (auf Basis Dibutylphthalat) und 3 Teile eines Emulgators auf Basis Sulfosuccinat (®Sultafon W 17210, Stockhausen GmbH, Krefeld) zu.

In 100 g dieser Losung gibt man 2 g des in Beispiel 3 dargestellten Produktes sowie 45 g Wasser. Man erhält eine monatelang lagerstabile Emulsion, die sich - selbst wenn sie sich trennen sollte - leicht durch Umschütteln homogenisieren läßt

Um diese Emulsion anwendungsfertig zu machen, verdünnt man sie 1:1 mit Wasser und rührt sie mit einem langsamtourigen Rührer oder einem Rührstab per Hand um.

Diese Emulsion wird auf ein grundiertes Leder gespritzt und im Aspekt mit einer handelsüblichen Celluloseacetobyrat-Emulsion (®EUSIN Emulsion EG, Bayer AG, Leverkusen) verglichen.

Beide überstanden 20 000 Naß-Knickungen im Bally-Flexometer (DIN 53351) ohne Beschädigung.

Die erfindungsgemäße Zurichtung überstand 200 000 Knickungen trocken, während die nach dem Stand der Technik leicht beschädigt war.

### Anwendungsbeispiel 4

### als Leimungsmittel für Papier

6,66 g eines Filterpapiers (Größe 24 x 24 cm) werden in der Leimpresse mit dem in Beispiel 6 hergestellten Produkt behandelt (Naßaufnahme 68 %). Nach dem Trocknen bei 100°C wurde der Cobb-Wert mit 81 bestimmt.

### Anwendungsbeispiel 4.1

100 g des in Beispiel 6 hergestellten Produktes wurden mit 50 g geschmolzenem Paraffin (Paraffin P 144, Wintershall, Düsseldorf) unter Rühren vermischt, 25 g heißes Wasser (ca. 80°C) zugefügt und unter Rühren abgekühlt. Es entstand eine 57 %ige wäßrige, stabile Dispersion.

6,7 g Papier wurden in der Leimpresse mit der 1:1 mit Wasser verdünnten, ober hergestellten, cremigen Emulsion behandelt. Naßaufnahme: 76 %. Nach dem Trocknen bei 100°C entstand ein geleimtes Papier mit einem Cobb-Wert von 20,7.

### Anwendungsbeispiel 5

150 g eines Kupferphthalocyaninblaus (Paliogenblau D 7080 der BASF, Ludwigshafen) werden und Dissolver mit 250 g des nach Beispiel 9 herausgestellten Dispergators, 592 g Wasser und 5 g Ammoniakwasser sowie 3 g Bentonit versetzt. Nach 20-minütigem Rühren wird in einer Perlmühle nachgemahlen. Es entstand eine blaue Pigmentanreibung.

### Anwendungsbeispiel 5.1

100 g PUR-Dispersion nach Beispiel 1 der DE-OS 41 33 572 werden mit 10 g der Pigmentdispersion aus Anwendungsbeispiel 5 versetzt.

Man fügt - unter der Beachtung, daß der pH-Wert der Mischung nicht unter 5 fällt, Verdickungsmittel zu. Dann druckt man die Mischung auf ein Baumwollgewebe und heizt bei 150°C aus.

Es entstand ein Textildruck, der sofort nach dem Trocknen 50 Naßbürstwäschen ohne Beschädigung überstand.

### Anwendungsbeispiel 6

Im Foulard wurde ein Baumwollgewebe mit 100 % Naßaufnahme des nach Beispiel 8 hergestellten Produkts (aus einer 30 g/l Lösung) behandelt Dann trocknete man 3 Minuten bei 120°C und 5 Minuten bei 150°C.

Das so ausgerüstete Gewebe erhielt durch diese Ausrüstung einen vollen, fettigen Griff, der auch nach 3-maligem Waschen bei 40°C noch spürbar blieb.

## Patentansprüche

1. Umsetzungsprodukte aus
a) organischem Polyisocyanat,
b) pro Äquivalent in a) enthaltenem NCO oder latentem NCO 0,005 bis 0,9 Äquivalente C₆-C₂₄-Alkohol,
c) pro Äquivalent in a) enthaltenem NCO oder latentem NCO 0,1 bis 0,995 Mol Cyanamid und
d) Ammoniak oder flüchtigem Amin als Neutralisationsmittel für die Cyanamidgruppen,
wobei diese Umsetzungsprodukte frei von Polyester- und Polyethergruppen und frei von Halogenatomen sind.

2. Produkte nach Anspruch 1, wonach als Komponente b) ein C₁₂-C₁₈-Alkohol eingesetzt worden ist.

3. Produkte nach Anspruch 1, wonach als Komponente a) Diisocyanate der Formeln III und/oder IV 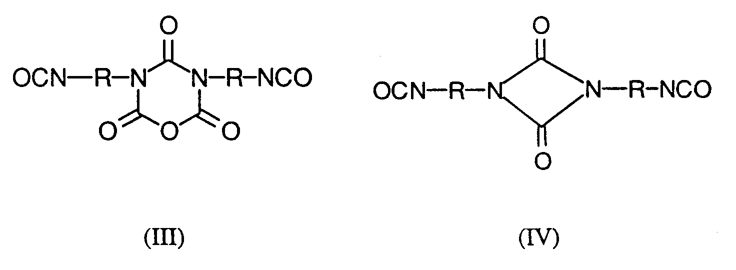 worin
R unabhängig voneinander für den zweiwertigen Rest eines aliphatischen Kohlenwasserstoffs mit 1 bis 15 C-Atomen, eines cycloaliphatischen Kohlenwasserstoffs mit 3 bis 15 C-Atomen, eines araliphatischen Kohlenwasserstoffs mit 7 bis 15 C-Atomen oder eines aromatischen Kohlenwasserstoffs mit 6 bis 12 C-Atomen steht,
verwendet werden.

4. Produkte nach Anspruch 1, wonach als Aufbaukomponente a) 3,5-Bis-(6-isocyanato-hexyl)-2,4,6-trioxotetrahydro-1,3,5-oxadiazin verwendet wird.

5. Verfahren zur Herstellung der Produkte nach Ansprüchen 1 bis 4 durch Umsetzung der Komponenten a) bis d).

6. Verwendung der Produkte nach Ansprüchen 1 bis 4 zur Nachgerbung von Leder, als Leimungsmittel für Papier, als Ausrüstmittel für Leder und Textilien und als Emulgatoren.

## Claims

1. Reaction products of
a) organic polyisocyanate,
b) from 0.005 to 0.9 equivalents of C₆-C₂₄ alcohol per equivalent of NCO or latent NCO contained in a),
c) from 0.1 to 0.995 mol cyanamide per equivalent of NCO or latent NCO contained in a) and
d) ammonia or volatile amine as neutralising agent for the cyanamide groups,
these reaction products being free from polyester groups and polyether groups and free from halogen atoms.

2. Products according to claim 1, for which a C₁₂-C₁₈ alcohol has been used as component b).

3. Products according to claim 1, for which diisocyanates corresponding to formulae III and/or IV 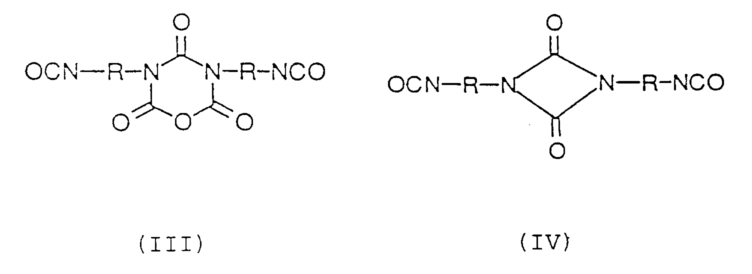 wherein
R independently of each other represent the divalent group of an aliphatic hydrocarbon having 1 to 15 C atoms, of a cycloaliphatic hydrocarbon having 3 to 15 C atoms, of an araliphatic hydrocarbon having 7 to 15 C atoms or of an aromatic hydrocarbon having 6 to 12 C atoms
are used as component a).

4. Products according to claim 1, for which 3,5-bis(6-isocyanatohexyl)-2,4,6-trioxotetrahydro-1,3,5-oxadiazine is used as the structural component a).

5. A process for the preparation of the products according to claims 1 to 4 by the reaction of components a) to d).

6. The use of the products according to claims 1 to 4 for the retanning of leather, as sizing agents for paper, as finishing agents for leather and textiles and as emulsifiers.

## Revendications

1. Produits réactionnels constitués par
a) un polyisocyanate organique,
b) par équivalent NCO contenu dans a) ou NCO latent, de 0,005 à 0,9 équivalent d'alcool en C₆-C₂₄,
c) par équivalent NCO contenu dans a) ou NCO latent, de 0,1 à 0,995 mole de cyanamide et
d) de l'ammoniac ou une amine volatile à titre d'agent de neutralisation pour les groupes cyanamide,
ces produits réactionnels étant exempts de groupes polyester et polyéther, et étant exempts d'atomes d'halogène.

2. Produits selon la revendication 1, pour lesquels on a mis en oeuvre à titre de composant b) un alcool en C₁₂-C₁₈.

3. Produits selon la revendication 1, pour lesquels, à titre de composant a), on utilise des diisocyanates répondant aux formules III et/ou IV 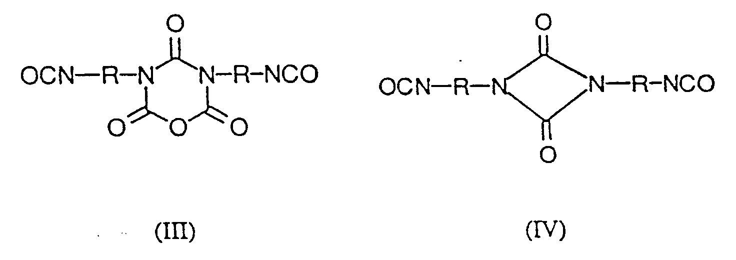 dans lesquelles
R représentent, indépendamment l'un de l'autre, le radical bivalent d'un hydrocarbure aliphatique contenant de 1 à 15 atomes de carbone, d'un hydrocarbure cycloaliphatique contenant de 3 à 15 atomes de carbone, d'un hydrocarbure araliphatique contenant de 7 à 15 atomes de carbone ou d'un hydrocarbure aromatique contenant de 6 à 12 atomes de carbone.

4. Produits selon la revendication 1, pour lesquels, à titre de constituant a), on utilise la 3,5-bis-(6-isocyanatohexyl)-2,4,6-trioxotétrahydro-1,3,5-oxadiazine.

5. Procédé pour la préparation des produits selon les revendications 1 à 4, par mise en réaction des composants a) à d).

6. Utilisation des produits selon les revendications 1 à 4, pour le retannage du cuir, à titre d'agent d'encollage pour le papier, à titre d'apprêt pour le cuir et les textiles, et à titre d'émulsifiants.
